# EUROPEAN PATENT APPLICATION

(11) **EP 2 145 952 A1**
(43) Date of publication of application: **20.01.2010**
(21) Application number: 08752532.5
(22) Date of filing: 09.05.2008
(51) Int. Cl.: C12N 5/06

(54) **METHOD OF DEGRADING BIOLOGICAL TISSUE**

(30) Priority: 11.05.2007 JP 2007126967
(71) Applicant: Olympus Corporation, Tokyo 151-0072 (JP); Cytori Therapeutics, Inc., San Diego, California 92121 (US)
(72) Inventor: TSUCHIDA, Hiroki, Tokyo 151-0072 (JP)
(74) Representative: von Hellfeld, Axel
(86) International application number: PCT/JP2008/058649
(87) International publication number: WO 2008/140046

(57) **Abstract**

A biological tissue is decomposed in a short time, and cells are obtained at a high yield without interfering with the health of the cells obtained through the decomposition. There is provided a method for decomposing biological tissue which decomposes a biological tissue under an action of a digestive enzyme, wherein a digestive juice containing the digestive enzyme is additionally supplied with the digestive enzyme either continuously or stepwisely over a predetermined time within a period from start of the decomposition until termination of the decomposition while the biological tissue is being immersed in the digestive juice.

## Description

### Technical Field

The present invention relates to a method for decomposing biological tissue.

### Background Art

Conventionally, methods for decomposing biological tissue are known in which a biological tissue is decomposed by immersing and agitating the biological tissue in a digestive juice containing digestive enzyme(s) (for example, refer to Patent Document 1).

Patent Document 1: PCT International Publication No. WO 2005/012480 Pamphlet

### Disclosure of Invention

However, enzymes in digestive juice come with disadvantages in that their activities decrease with the progress of the decomposition of biological tissues and that the decomposition efficiency decreases. If the decomposition is made progress in this state, a long time is required to complete the decomposition of biological tissues, and a problem is involved in that the yield of cells obtained through the decomposition can not be increased.

The present invention takes the above problems into consideration, with an object of providing a method for decomposing biological tissue capable of decomposing a biological tissue in a short time and obtaining cells at a high yield without interfering with the health of the cells obtained through the decomposition.

The present invention employs the following means in order to achieve the above object.
The present invention provides a method for decomposing biological tissue which decomposes a biological tissue under an action of a digestive enzyme, wherein a digestive juice containing the digestive enzyme is additionally supplied with the digestive enzyme either continuously or stepwisely over a predetermined time within a period from start of the decomposition until termination of the decomposition while the biological tissue is being immersed in the digestive juice.

According to the present invention, when the decomposition is initiated by immersing and agitating a biological tissue in a digestive juice, the biological tissue is decomposed to thereby make cells float in the digestive juice. Then, the activity of the digestive enzyme decreases with the progress of the decomposition. Therefore, by additionally supplying the digestive enzyme to the digestive juice either continuously or stepwisely within the period from start of the decomposition until termination of the decomposition, the activity of the digestive enzyme in the digestive juice can be maintained and the efficiency of decomposing the biological tissue can be improved. By so doing, the cell yield can be improved as compared to a case where the digestive enzyme is put into the digestive juice at the beginning to decompose the biological tissue without any subsequent addition.

The above invention may also comprise: a total amount determination step of determining a total amount of the digestive enzyme required for completing the decomposition of the biological tissue; and an enzyme supply step of additionally supplying the digestive enzyme so that the determined total amount of the digestive enzyme can be supplied from start of the decomposition of the biological tissue until termination of the decomposition.
By so doing, since the total amount of the digestive enzyme determined by the total amount determination step is supplied from start of the decomposition until termination of the decomposition, cells can be kept from being exposed to a high concentration of the enzyme immediately after the start of the decomposition. Further, more than necessary amount of the digestive enzyme is not added, thereby preventing the digestive enzyme having a high activity from acting on cells after the termination of the decomposition, and the health of the cells can be maintained.

Moreover, in the above invention, the enzyme supply step may also comprise: an activity estimation step of estimating an activity of the digestive enzyme; a judgment step of judging whether or not the estimated activity of the digestive enzyme is in a decreasing tendency; and an additional supply step of additionally supplying the digestive enzyme if the result of the judgment shows that the estimated activity of the digestive enzyme is in a decreasing tendency.
By so doing, if the judgment step judges that the activity of the digestive enzyme estimated by the activity estimation step is in a decreasing tendency, the digestive enzyme is additionally supplied by the additional supply step, and thus the biological tissue can be decomposed while maintaining the activity of the digestive enzyme at a high state. As a result, the efficiency of decomposing the biological tissue can be improved and the cell yield can be increased.

Furthermore, in the above invention, the activity estimation step may also comprise: a density measurement step of measuring a cell density in a cell suspension containing the digestive enzyme; and a density change calculation step of calculating a timewise change of the measured cell density, so that the judgment step judges that the activity of the digestive enzyme is in a decreasing tendency at a time point at which the calculated timewise change of the cell density reaches an approximate local maximum.
By so doing, by measuring the cell density in the cell suspension by the density measurement step and calculating the timewise change of the cell density by the density change calculation step, the judgment step can judge that the activity of the digestive enzyme is in a decreasing tendency at a time point at which the timewise change of the cell density reaches to a local maximum. Although the activity per se of the digestive enzyme is difficult to detect, the activity of the digestive enzyme can be readily estimated by using the timewise change of the cell density.

In the above invention, the digestive juice may comprise a mixed solution of the digestive enzyme with either a Ringer's solution or a phosphate buffer solution.
By so doing, the biological tissue is decomposed by a mixed solution of the digestive enzyme with either a Ringer's solution or a phosphate buffer solution adjusted to near-neutral pH. Therefore, lowering in the survival rate of cells obtained after the decomposition can be prevented.

The present invention demonstrates effects of enabling to decompose a biological tissue in a short time and to obtain cells at a high yield without interfering with the health of the cells obtained through the decomposition.

### Brief Description of Drawings

[FIG. 1] A flowchart showing a method for decomposing biological tissue according to one embodiment of the present invention.
[FIG. 2] A flowchart showing the activity estimation step in the method for decomposing biological tissue of FIG. 1.
[FIG. 3] A graph showing a timewise change of the cell density in a digestive juice in the decomposition step of the method for decomposing biological tissue of FIG. 1.
[FIG. 4] A graph showing the first derivative of the timewise change of the cell density of FIG. 2.
[FIG. 5] A graph showing the second derivative of the timewise change of the cell density of FIG. 2.
[FIG. 6] A graph showing the timewise change of the cell density when the digestive enzyme is additionally supplied to the digestive juice in mid course of the decomposition step of the method for decomposing biological tissue of FIG. 1.
[FIG. 7] A graph showing cell yields obtained in one embodiment of the method for decomposing biological tissue of FIG. 1, with comparison to a conventional decomposition method.

### Explanation of Reference Signs

- S1:: Total amount determination step
- S4:: Activity estimation step
- S5:: Judgment step
- S6:: Additional supply step
- S31:: Enzyme supply step
- S41:: Density measurement step
- S42:: Density change calculation step

### Best Mode for Carrying Out the Invention

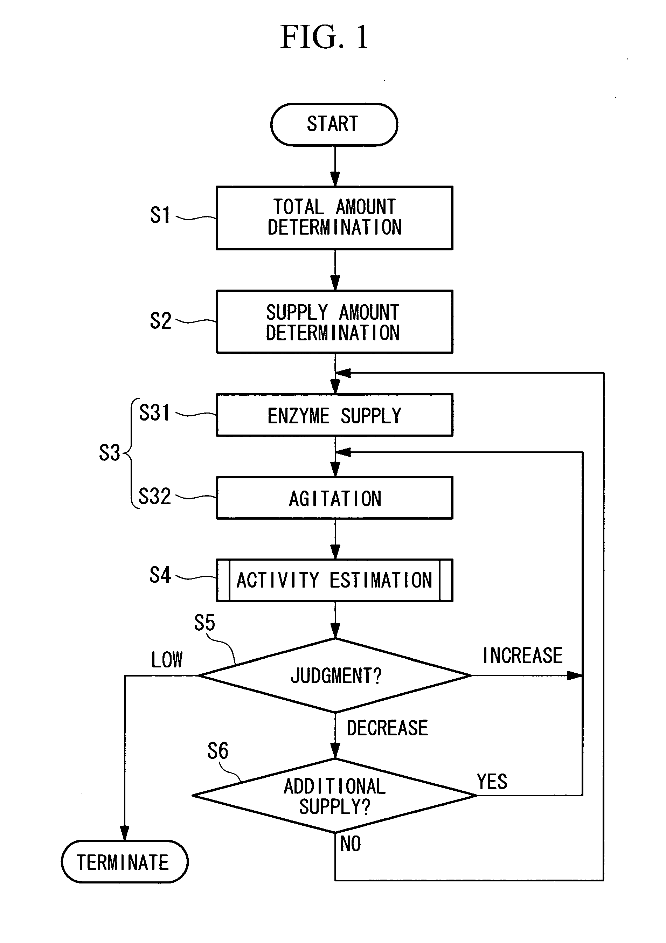

Hereunder is a description of a method for decomposing biological tissue according to one embodiment of the present invention, with reference to FIG. 1 to FIG. 7.
The method for decomposing biological tissue according to the present embodiment is, for example, a method for decomposing adipose tissue, which comprises as shown in FIG. 1: a total amount determination step S1 of determining a total amount of a digestive enzyme required for completing the decomposition; a supply amount determination step S2 of determining a supply amount of the digestive enzyme; a decomposition step S3 of decomposing the biological tissue in a digestive juice supplied with the digestive enzyme; an activity estimation step S4 of estimating an activity of the digestive enzyme; a judgment step S5 of judging the estimated activity of the digestive enzyme; and an additional supply step S6 of judging based on the judgment result whether or not the total amount of the digestive enzyme has been completely supplied, and additionally supplying the digestive enzyme in cases of incomplete supply.

In the total amount determination step S1, the total amount of the digestive enzyme required for completing the decomposition is determined based on the total weight or volume of an adipose tissue to be decomposed. In the supply amount determination step S2, thus determined total amount of the digestive enzyme is divided into a plurality of portions to determine an amount of the digestive enzyme to be supplied first and amount(s) of the digestive enzyme to be additionally supplied thereafter. The decomposition step S3 comprises: an enzyme supply step S31 of mixing a Ringer's solution with the digestive enzyme at the amount to be supplied first that has been determined in the supply amount determination step S2; and an agitation step S32 of immersing and agitating the adipose tissue in the prepared digestive juice.

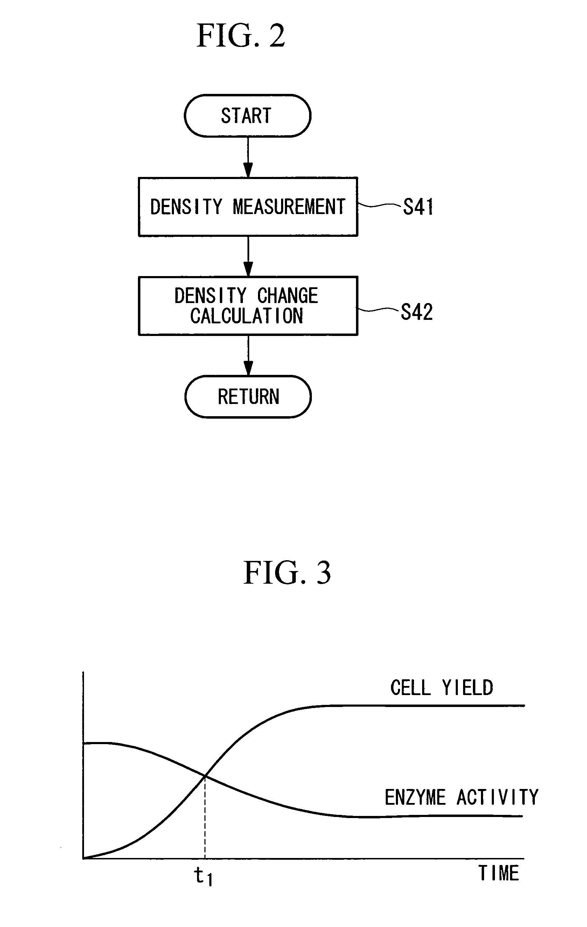

As shown in FIG. 2, the activity estimation step S4 comprises: a density measurement step S41 of sampling the digestive juice at predetermined time intervals and measuring the number of cells floating in the digestive juice to thereby measure a cell density in the digestive juice; and a density change calculation step S42 of calculating a timewise change of the cell density measured in the density measurement step S41. As shown in FIG. 3, since the number of cells separated from the biological tissue and floating in the digestive juice increases with the progress of the decomposition of the biological tissue, the cell density increases. Meanwhile, the activity of the digestive enzyme decreases.

As a result, looking at the timewise change of the cell density, the degree of the increase is not constant, but the degree of the increase of the timewise change of the cell density increases within a predetermined time after the start of the decomposition, then slows down after a certain time point t1, and thereafter decreases with the passage of time.

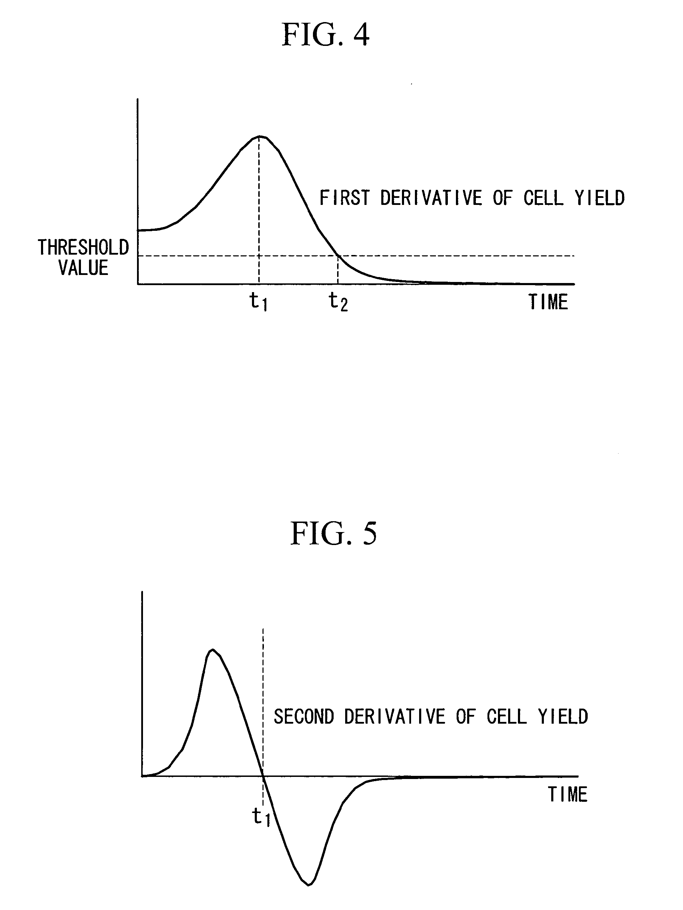

FIG. 4 is a graph showing the first derivative of the timewise change of the cell density shown in the graph of FIG. 3, with respect to time. FIG. 5 is a graph showing the second derivative thereof. That is to say, according to these graphs, the amount of increased number of cells per unit time (timewise change of the cell density) turns from an increasing tendency into a decreasing tendency at the time point t1.

Therefore, in the judgment step S5, the time point t1 at which the timewise change of the cell density in FIG. 4 reaches the local maximum value, specifically at which the second derivative of the timewise change of the cell density in FIG. 5 becomes approximately zero, is judged, so that the digestive enzyme at a previously determined additional supply amount can be additionally supplied into the digestive juice in the additional supply step. Moreover, in the judgment step, a time point t2 at which the increased amount of the timewise change of the cell density in FIG. 4 becomes approximately zero (falls below a threshold value) is judged, so that the decomposition treatment on the biological tissue by the decomposition step S3 can be terminated.

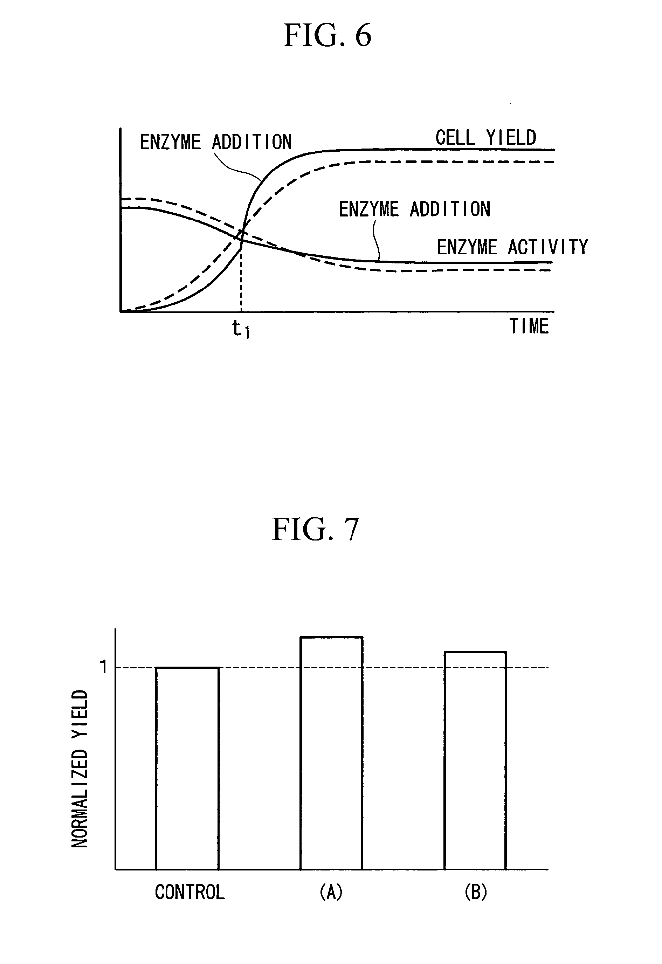

According to thus constituted method for decomposing biological tissue according to the present embodiment, the digestive enzyme is additionally supplied at the time point t1 at which the second derivative of the timewise change of the cell density becomes approximately zero. Therefore, as shown in FIG. 6, unlike the curved line of the timewise change before the additional supply of the digestive enzyme shown by the broken line, the timewise change of the cell density is altered as shown by the solid line along a new curve made by shifting the increased amount of the timewise change of the cell density toward a further increasing tendency. As a result, an advantage is provided in which the number of obtained cells, namely the cell yield, can be improved.

In this case, the activity per se of the digestive enzyme for which the measurement takes time, is not used, but the activity of the digestive enzyme is estimated by using the timewise change of the cell density. Therefore, the timing for additionally supplying the digestive enzyme can be determined in real time. As a result, the biological tissue can be more efficiently decomposed, the time required for the decomposition can be shortened, and the cell yield can be improved.

Moreover, since the additional supply of the digestive enzyme accelerates the decomposition of the biological tissue, an advantage is also provided in which the biological tissue can be decomposed in a short time.
Further, according to the method for decomposing biological tissue according to the present embodiment, the total amount of the digestive enzyme to be supplied to the digestive juice until completion of the decomposition is previously determined prior to the start of the decomposition based on the weight or volume of the biological tissue, and does not exceed the determined value. Accordingly, an advantage is further provided in which decomposed cells can be kept from being in contact with the digestive enzyme having a remaining high activity, to thereby improve the health of the obtained cells.

FIG. 7 shows cell yields (A) and (B) obtained through a first supply of three-quarters of the total amount of the digestive enzyme required for completing the decomposition of the biological tissue and an additional supply of the remaining one-quarter thereof in mid course of the decomposition, with comparison to a conventional case (control) where the total amount of the digestive enzyme was entirely added at the beginning. According to this, the cell yield was found to be increased by 5 to 15% as compared to the conventional method. Moreover, the time required for completing the decomposition was also found to be shortened.

In the present embodiment, the total amount of the digestive enzyme required for completing the decomposition was divided to be additionally supplied for a plurality of times. However, instead of this, additional supply may be continuously performed within a range of the total amount of the digestive enzyme over a predetermined time.

Moreover, in the present embodiment, the timing for additionally supplying the digestive enzyme is determined by detecting the time point t1 at which the second derivative of the timewise change of the cell density becomes approximately zero. However, the judgment at this time point t1 is not strictly required. For example, the digestive enzyme may be additionally supplied at any time point before or after the time when the timewise change of the cell density turns into a decreasing tendency, or the digestive enzyme may also be additionally supplied when the timewise change of the cell density reaches a predetermined value.

## Claims

1. A method for decomposing biological tissue which decomposes a biological tissue under an action of a digestive enzyme,
wherein a digestive juice containing the digestive enzyme is additionally supplied with the digestive enzyme either continuously or stepwisely over a predetermined time within a period from start of the decomposition until termination of the decomposition while the biological tissue is being immersed in the digestive juice.

2. A method for decomposing biological tissue according to claim 1, which comprises: a total amount determination step of determining a total amount of the digestive enzyme required for completing the decomposition of the biological tissue; and
an enzyme supply step of additionally supplying the digestive enzyme so that the determined total amount of the digestive enzyme can be supplied from start of the decomposition of the biological tissue until termination of the decomposition.

3. A method for decomposing biological tissue according to claim 2, wherein said enzyme supply step comprises:
an activity estimation step of estimating an activity of the digestive enzyme;
a judgment step of judging whether or not the estimated activity of the digestive enzyme is in a decreasing tendency; and
an additional supply step of additionally supplying the digestive enzyme if the result of the judgment shows that the estimated activity of the digestive enzyme is in a decreasing tendency.

4. A method for decomposing biological tissue according to claim 3, wherein the activity estimation step comprises: a density measurement step of measuring a cell density in a cell suspension containing the digestive enzyme; and a density change calculation step of calculating a timewise change of the measured cell density, and
said judgment step judges that the activity of the digestive enzyme is in a decreasing tendency at a time point at which the calculated timewise change of the cell density reaches an approximate local maximum.

5. A method for decomposing biological tissue according to any one of claim 1 through claim 4, wherein the digestive juice comprises a mixed solution of the digestive enzyme with either a Ringer's solution or a phosphate buffer solution.
